(19) Europäisches Patentamt

European Patent Office

Office européen des brevets

(11) Veröffentlichungsnummer: **0 418 992 A2**

(12) **EUROPÄISCHE PATENTANMELDUNG**

(21) Anmeldenummer: 90250241.8

(22) Anmeldetag: 20.09.90

(51) Int. Cl.⁵: **A61B 3/16**

(30) Priorität: 20.09.89 DE 3931821

(43) Veröffentlichungstag der Anmeldung:
27.03.91 Patentblatt 91/13

(84) Benannte Vertragsstaaten:
**DE FR GB**

(71) Anmelder: **Obal, Adalbert, Dr.**
**Mansfelder Strasse 15**
**W-1000 Berlin 31(DE)**

(72) Erfinder: **Obal, Adalbert, Dr.**
**Mansfelder Strasse 15**
**W-1000 Berlin 31(DE)**

(74) Vertreter: **Christiansen, Henning, Dipl.-Ing.**
**Patentanwalt CHRISTIANSEN Pacelliallee**
**43/45**
**W-1000 Berlin 33(DE)**

(54) **Impressionstonometer.**

(57) Impressionstonometer zur Messung des Augeninnendruckes und der Rigidität des Augapfels auf der Basis impressionstonometrischer Messungen mit fixierbarem Meßstempel, dessen relative Position zu einer Bezugsfläche ein Maß für den Augeninnendruck bildet und einem elektronischen bzw. mechanischen Tiefentaster, das aus einem im wesentlichen zylinderförmigen Handgerät mit zwei einander gegenüberliegenden fixierbaren Meßstempeln besteht, welche unterschiedlichen axialen Auflagekräften ausgesetzt sind sowie einer Aufnahmevorrichtung mit einer zylindrischen Führung, wobei am Ende der zylindrischen Führung der Tiefentaster mit von außen ablesbaren Anzeigevortungen für den Augeninnendruck und die Rigidität angeordnet ist.

Fig.1

## IMPRESSIONSTONOMETER

Die Erfindung betrifft ein Impressionstonometer der im Oberbegriff des Anspruchs 1 angegebenen Art.

Bekannt sind Impressionstonometer, die der Messung des Augeninnendruckes dienen. Ein Impressionstonometer mit fi xierbarem Meßstempel ist beispielsweise in der DE-OS 37 18 689 beschrieben. Bei diesem Gerätetyp erfolgen der Meßvorgang und die Ablesung der Meßwerte zeitlich und örtlich getrennt voneinander. Die beim Aufsetzen des Gerätes auf die Hornhaut entgegen einer Kraft, insbesondere Federkraft bewirkte Verschiebung eines Stempels ist ein Maß für den Augeninnendruck. Nach dem Fixieren der Stellung des Stempels erfolgt die Messung des Verschiebeweges und damit des Augeninnendruckes. Bisher wurden derartige Wegmessungen vorwiegend mit getrennten Meßwerkzeugen, vorzugsweise mit einer Meßuhr oder einer Mikrometerschraube durchgeführt. Nachteilig ist dabei vor allem die relativ umständliche Handhabung derartiger Geräte. Außerdem sind zur Ermittlung des als Rigidität bezeichneten Widerstandes des Augapfels gegen Dehnung Messungen mit Impressionstonometern unterschiedlicher Stempelauflagekräfte, vorzugsweise 53,9 mN und 98,1 mN, gegen die der Meßstempel in unterschiedlichem Ausmaß verschoben wird, erforderlich. Zur Bestimmung der Rigidität dient ein Nomogramm, in welchem Meßwertkurven und die Rigiditätskurve abgetragen sind. Durch das Verbinden der beiden Meßwerte in dem Monogramm und Parallelverschieben dieser Geraden ergibt sich der Rigiditätswert. Eine derartige Methodik ist nicht nur zeitaufwendig und umständlich sondern auch in hohem Maße ungenau.

Der Erfindung liegt die Aufgabe zugrunde, ein kombiniertes Gerät zur Messung des Augeninnendruckes und der Rigidät des Augapfels auf der Basis impressionstonometrischer Messungen anzugeben, wobei insbesondere vereinfachte Handhabung und erhöhte Meßgenauigkeit anzustreben sind.

Diese Aufgabe wird mit den kennzeichnenden Merkmalen des Anspruchs 1 gelöst.

Die Erfindung beruht auf der Erkenntnis, daß ein weitgehend zylinderförmiges Handgerät mit zwei gegenüberliegenden fixierbaren Meßstempeln, die mit unterschiedlichen Auflagekräften belastet sind, nacheinander mit beiden Meßstempeln in eine mit Auswerte- und Anzeigeeinheiten versehen Aufnahmevorrichtung einschiebbar ist, wobei die Meßgröße "Weg" als Verschiebung des Fühlelements eines in der Aufnahme für das Handgerät vorgesehenen, vorzugsweise elektromechanischen, Wandlers vorgesehen ist.

Entsprechend einer vorteilhaften Weiterbildung der Erfindung kann das Handgerät aus zwei füllhalterähnlichen Handgeräten, die jeweils verschieden vorgespannte Meßstempel aufweisen, zusammengesetzt werden. Dadurch erhöht sich die Anzahl der verwendbaren Meßstempelkombinationen.

Um eine exakt reproduzierbare Position des Impressionstonometers relativ zu der Aufnahme zu gewährleisten, ist die Aufnahmevorrichtung mit einer zylindrischen Führung und einem Anschlag versehen. Innerhalb der Aufnahmevorrichtung ist ein Tiefentaster vorgesehen, der beim Einschieben des Impressionstonometers in Abhängigkeit von der fixierten Meßstempelstellung auswertbare Veränderungen erfährt. Die Ablesung des Wertes für den Augeninnendruck erfolgt mittels einer außen an dem Aufnahmezylinder vorgesehenen Anzeigevorrichtung.

Der Vorteil einer derartigen Meßvorrichtung besteht insbesondere darin, daß keinerlei manuelles Einrichten der Meßmittel erforderlich ist.

Entsprechend einer vorteilhaften Weiterbildung der Erfindung ist der Tiefentaster mit einem resistiven kapazitiven oder induktiven Wegaufnehmer versehen, welcher Bestandteil eines Oszillators oder eines Schwingkreises ist. Entsprechend dem Betrag der Änderung eines Widerstandes, einer Kapazität oder einer Induktivität verändert sich auch die Oszillator- bzw. Schwingkreisfrequenz, welche relativ einfach und sehr genau meßbar bzw. auswertbar ist. Eine sich anschließende Anzeigevorrichtung dient der direkten Anzeige des Augeninnendruckes.

Vorzugsweise ist der Tiefentaster mit einem kapazitiven Wegaufnehmer ausgestattet welcher eine gegen eine Federkraft verschieblich gelagerte Kondensatorplatte aufweist. Der fixierte Meßstempel verschiebt beim Einführen des Impressionstonometers in die Aufnahmevorrichtung die Kondensatorplatte bzw. einen mit der Kondensatorplatte verbundenen Sensor oder Fühlstift, wodurch die mechanische in eine elektrische Meßgröße umgewandelt wird. Die resultierende Änderung der Schwingkreis- oder Oszillatorfrequenz wird mittels einer Meßschaltung in digitale Ausgangssignale überführt, welche mit dem Adresseneingang eines nachgeschalteten Festwertspeichers verbunden sind. Der Festwertspeicher ordnet den Ausgangssignalen der Meßschaltung tabellenartig diejenigen Werte für den Augeninnendruck zu, die der Position des Meßstempels entsprechen. Diese Werte sind digital anzeigbar. Dazu dient insbesondere ein Sieben-Segment-Display bzw. ein LCD-Display.

Der Beginn des Meßvorganges wird vorzugsweise durch die automatische Betätigung eines Schalters während des Ein führens des Impressionstonometers in den Aufnahmezylinder ausgelöst. Der Schalter ist mittels eines Zeitgliedes nach einem vorgegebenen Zeitraum, beispielsweise 20 Sekunden in den Aus-Zustand versetzbar. Nach dieser Zeit erlischt die digitale Anzeige. Durch kurzes Herausziehen und Wiedereinsetzen des Impressionstonometers in die Aufnahmevorrichtung wird eine erneute 20 Sekunden währende Meßwertanzeige ausgelöst.

Zur Ermittlung des Rigiditätswertes ist die Verknüpfung zweier bei unterschiedlichen Stempeldrucken gemessenen Stempelverschiebungen erforderlich. Dementsprechend ist das Handgerät mit zwei vorzugsweise einander gegenüberliegenden fixierbaren Meßstempeln versehen. Die beiden Stempeldruckwerte betragen üblicherweise 53,9 mN und 98.1 mN. Die zur Ermittlung des Rigiditätswertes gegenüber der Druckermittlung erforderliche zusätzliche Messung erfolgt durch erneutes Einsetzen des umgedrehten Handgerätes in die zylindrische Führung der Aufnahmevorrichtung, wobei durch das Einschieben der erste und zusätzlich ein zweiter Schalter betätigt wird, der die Meßschaltung derart aktiviert, daß ein zweiter Festwertspeicher angesteuert wird. Dieser ist bereits mit Meßdaten aus der vorangegangenen Druckermittlung adressiert. Die hinzukommenden Meßdaten aus der zweiten Messung vervollständigen die Adressierung. Die Verknüpfung der Meßdaten erfolgt nach dem gleichen Prinzip wie die Ermittlung des Augeninnendruckes, nämlich durch tabellenartige Zuordnung des Rigiditätswertes zu einem bestimmten Meßdatenpaar. Ein nachgeschalteter Decoder und ein Display ermöglichen die Ablesung des Rigiditätswertes.

Der Stromverbrauch einer derartigen Meßschaltung, die nach einer bestimmten Zeit automatisch unterbrochen wird, ist außerordentlich gering, so daß als Energiequelle Batterien oder Akkumulatoren einsetzbar sind.

Das erfindungsgemäße Impressionstonometer ermöglicht durch die Messung der Stempelverschiebungen sowohl die Ermittlung des Augeninnendruckes als auch der Rigidität des Augapfels. Das Gerät bzw. die Anwendung des Gerätes zeichnet sich vor allem durch geringen Zeitaufwand, hohe Präzision und digitale Anzeige aus.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine Frontalansicht eines erfindungsgemäßen Impressionstonometers in teilweise geschnittener Darstellung,

Figur 2 eine Aufnahmevorrichtung als Detail der Impressionstonometers gemäß Figur 1 in perspektivischer Darstellung,

Figur 3 eine schematische, teilweise geschnittene Darstellung eines in einer Aufnahmevorrichtung eingesetzten fixierten Meßstempels und

Figur 4 ein Blockschaltbild einer Meßanordnung auf der Grundlage eines kapazitiven Wegaufnehmers.

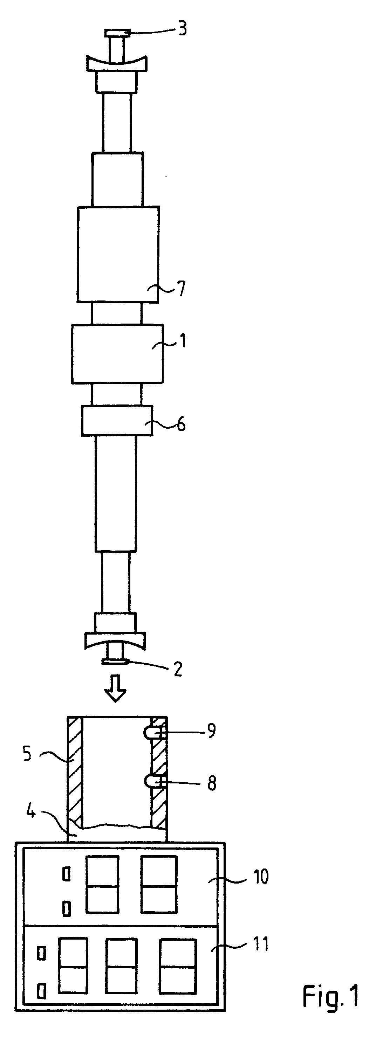

Das in Figur 1 dargestellte Impressionstonometer besteht im wesentlichen aus einem weitgehend zylinderförmigen Handgerät 1 zur Einstellung und Fixierung zweier an entgegengesetzten Enden des Handgerätes 1 befindlicher Meßstempel 2 und 3 sowie einer Aufnahmevorrichtung 4 zur Messung und Auswertung der Stempelverschiebungen. Die Aufnahmevorrichtung 4 ist mit einer zylindrischen Führung 5 versehen, die zu dem Durchmesser zweier zylinderförmiger Abschnitte 6 und 7 des Handgerätes 1 gepaßt ist, wodurch zum einen das nacheinander erfolgende Einschieben beider Stempelenden des Handgerätes 1 in die zylindrische Führung 5 der Aufnahmevorrichtung 4 erleichtert wird und zum anderen in Abhängigkeit von der Einschiebrichtung entweder nur der Kontakt 9 oder die beiden Kontakte 8 und 9 geschlossen werden. Die Kontakte 8 und 9 dienen der Ansteuerung der weiter unten beschriebenen Auswerteelektronik. Der Augeninnendruck und die Rigidität des Augapfels sind mittels Anzeigeeinheiten 10 und 11 direkt an der Aufnahmevorrichtung 4 ablesbar.

Gemäß einer nicht dargestellten Weiterbildung kann das Handgerät 1 auch aus zwei füllhalterähnlichen Handgeräten, die jeweils verschieden vorgespannte Meßstempel aufweisen, zusammengesetzt werden.

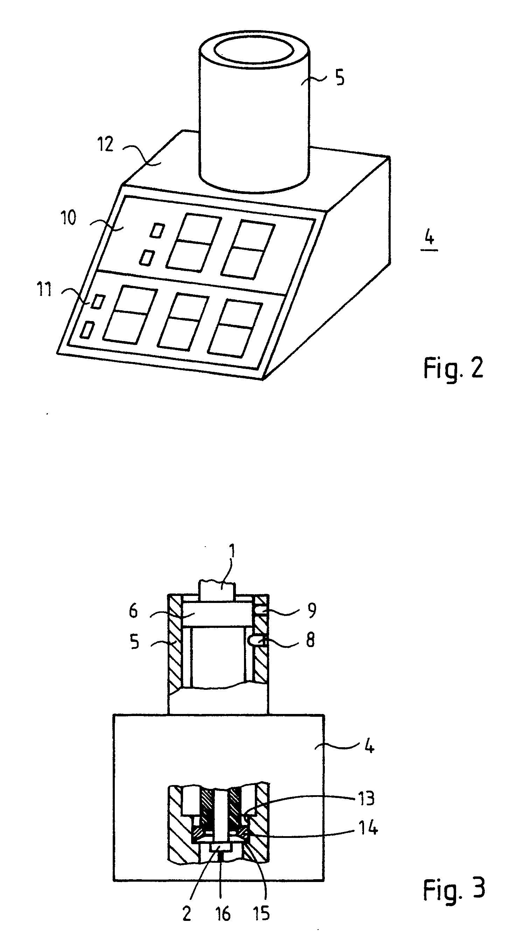

Die Figur 2 zeigt die Aufnahmevorrichtung 4 in einer perspektivischen Ansicht. Die zylindrische Führung 5 ist auf einem Standsockel 12 aufgesetzt, welcher die Meßschaltung aufnimmt und an einer gut sichtbaren Stelle seiner äußeren Peripherie mit den Anzeigeeinheiten 10 und 11 versehen ist.

In der Figur 3 ist die Lage des in der Aufnahmevorrichtung 4 eingeschobenen Handgerätes 1 dargestellt. Zur Zentrierung des Handgerätes 1 innerhalb der Aufnahmevorrichtung 4 ist neben der zylindrischen Führung 5 eine weitere zylindrische Führung 13 vorgesehen, die dem Durchmesser der Fußplatte 14 des Handgerätes 1 angepaßt ist. Die Fußplatte 14 wird zum Zwecke des Einstellens des Handgerätes 1 mit einem bestimmten Druck auf die Hornhaut des Auges aufgesetzt, während der Meßstempel 2 bzw. 3 durch eine zentrische Bohrung der Fußplatte 14 mit etwas höherem Druck die Hornhaut eindellt. Der Grad der Eindellung und damit die Verschiebung des Stempels 2 bzw. 3 gegenüber der Fußplatte 14 ist ein Maß für den Augeninnendruck. Diese Stellung wird mittels eines Hebelmechanismus an dem Handgerät 1 fixiert und durch das Einschieben in die Aufnahmevorrichtung 3 ermittelt. Dazu ist ein ringförmiger Anschlag 15 für die Fußplatte 14 innerhalb der Aufnahmevorrichtung 4 vorgesehen, welcher die Einschiebtiefe des Handgerätes 1 begrenzt. Der Meßstempel 2 bzw. 3 ragt durch den ringförmigen Anschlag 15 hindurch und verschiebt einen Fühlstift 16, der mit einem mechanischen oder elektronischen Tiefentaster verbunden ist.

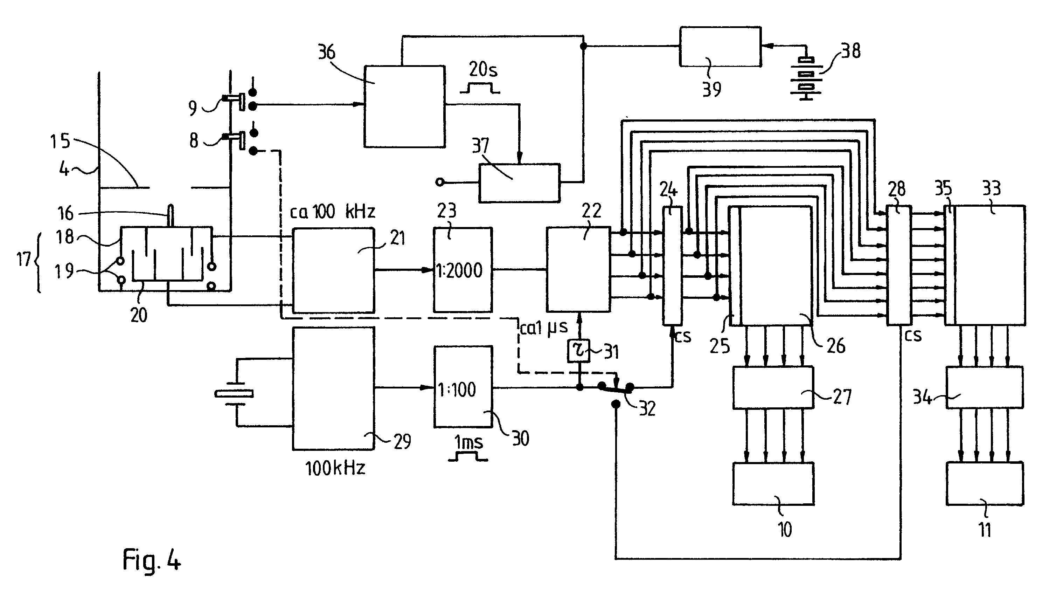

In der Figur 4 ist ein Ausführungsbeispiel eines elektronischen Tiefentasters anhand eines Blockschaltbildes dargestellt. Der verschiebliche Fühlstift 16 betätigt einen kapazitiven Wegaufnehmer 17, indem mit dem Fühlstift 16 eine Kondensatorplatte 18 mitbewegt wird. Um eine reproduzierbare Ausgangsstellung der verschieblichen Kondensatorplatte 18 bzw. des Fühlstiftes 16 zu gewährleisten, ist eine Rückstellfeder 19 zwischen der Platte 18 und einem Gehäuseelement der Aufnahmevorrichtung 4 vorgesehen. Entsprechend der Verringerung des Plattenabstandes der verschieblichen Kondensatorplatte 18 gegenüber einer festen Platte 20 vergrößert sich die Kapazität des Kondensators. Als Bestandteil eines Oszillators 21 beeinflußt der Kondensator somit die Frequenz des Oszillators 21. Diese Frequenz ist ein Maß des zu bestimmenden Augeninnendruckes. Die Messung der Oszillatorfrequenz erfolgt mittels eines Zählers 22, der sich einem dem Oszillator 21 nachgeschalteten Frequenzteiler 23 anschließt. Das Ausgangssignal des Zählers 22 ist über einen ersten als Latch 24 ausgebildeten Zwischenspeicher mit dem Adresseneingang 25 eines ersten ROM-Festwertspeichers 26 verbunden, wobei ein dem Zählerstand entsprechender Adreßwert angesprochen wird, dem nach Art einer Tabelle ein bestimmtes Ausgangssignal zugeordnet ist. Der in dem adressierten Speicher enthaltene Wert wird als ROM-Ausgangssignal mittels eines nachgeschalteten ersten Decoders 27 demoduliert und einer digitalen Anzeigeeinheit 23 zur Anzeige des Augeninnendruckes zugeführt.

Die Ausgangssignale des ersten Latch 24 werden neben dem ersten Festwertspeicher 26 auch einem zweiten Latch 28 zugeführt und für die Weiterverarbeitung im Zusammenhang mit der Rigiditätsermittlung gespeichert.

Zur zeitlichen Steuerung des Zählvorganges und zur Aktivierung jeweils eines der beiden Latches 24 und 28 mittels "chip-select" ist ein Schaltungszweig mit einem Taktgeber 29 vorgesehen, dem ein weiterer Frequenzteiler 30 nachgeschaltet ist. Diese Schaltungsanordnung steuert die Speicher-Latches 24 und 28 mit CS-Befehlen derart, daß zum Beispiel im Abstand von jeweils 1 ms ein Meßwert ermittelt und codiert wird. Gleichzeitig ist der Zähler 22 über ein "Reset-Glied" 31 so angesteuert, daß zwischen den Meßwertermittlungen jeweils eine Zählpause von beispielsweise 1 µs liegt.

In Abhängigkeit von der Einsetzrichtung des Handgerätes 1 in die Aufnahmevorrichtung 4 wird entweder nur der Kontakt 9 betätigt oder die Kontakte 8 und 9 werden betätigt. Beim Schließen des Kontaktes 9 wird nur das erste Latch 24 angesteuert, wodurch die Ermittlung und die Anzeige des Augeninnendruckes ausgelöst wird. Durch Umdrehen des Handgerätes 1 und erneutes Einschieben in die zylindrische Führung 5 der Aufnahmevorrichtung 4 werden die Kontakte 8 und 9 geschlossen und - beispielsweise über ein Relais -gleichzeitig der Schalter 32 betätigt. Das hat zur Folge, daß nunmehr das zweite Latch 28 und damit der zweite ROM-Festwertspeicher 33 und der zweite Decoder 34 angesteuert werden. Der zweite ROM-Festwertspeicher 33 hat die Aufgabe, die der Verschiebung beider Meßstempel entsprechenden Signale den entsprechenden Rigiditätswerten zuzuordnen und über den zweiten Decoder 34 der Anzeigeeinheit 11 zuzuführen. Die Daten der ersten Messung sind bereits im Adresseneingang 35 des zweiten Speichers 33 abgelegt. Dazu ist es erforderlich, daß immer zuerst der für die Ermittlung des Augeninnendruckes vorgesehene Meßstempel in die zylindrische Führung 5 der Aufnahmevorrichtung 4 eingesetzt wird. Andernfalls erfolgt keine Anzeige, da der Adresseneingang 35 des Speichers 33 nicht vollständig besetzt ist.

Ein separater Schaltungszweig mit einem als Monoflop ausgebildeten Zeitglied 36 bewirkt, daß der Meßschaltkreis nach einer vorgegebenen Meßzeit unterbrochen wird. Der von dem Monoflop nach einem bestimmten Zeitraum abgegebene Impuls betätigt einen Schalter 37, der mit der von einer Batterie 38 gespeisten Stromversorgungseinrichtung 39 verbunden ist und den Stromkreis unterbricht.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Ansprüche**

1. Impressionstonometer zur Messung des Augeninnendrukkes und der Rigidität des Augapfels auf der Basis impressionstonometrischer Messungen mit fixierbarem Meßstempel, dessen relative Position zu einer Bezugsfläche ein Maß für den Augeninnendruck bildet und einem elektronischen bzw. mechanischen Tiefentaster,
**gekennzeichnet durch**
ein im wesentlichen zylinderförmiges Handgerät (1) mit zwei einander gegenüberliegenden fixierbaren Meßstempeln (2 und 3), welche unterschiedlichen axialen Auflagekräften ausgesetzt sind sowie einer Aufnahmevorrichtung (4) mit einer zylindrischen Führung (5), wobei am Ende der zylindrischen Führung (5) der Tiefentaster mit von außen ablesbaren Anzeigevorrichtungen (10 und 11) für den Augeninnendruck und die Rigidität angeordnet ist.

2. Impressionstonometer nach Anspruch 1 , **gekennzeichnet durch** seine Verwendung als Differentialtonometer zur Bestimmung der Rigidität.

3. Impressionstonometer nach einem der vorangehenden Ansprüche, **dadurch gekennzeichnet,** daß die unterschiedlichen axialen Auflagekräfte, die auf die Meßstempel wirken, 53,9 mN und 98,1 mN betragen.

4. Impressionstonometer nach einem der vorangehenden Ansprüche, **dadurch gekennzeichnet,** daß die Aufnahmevorrichtung (4) einen Ständer oder eine Umhüllung zum Schutz des Handgerätes (1) bildet.

5. Impressionstonometer nach einem der vorangehenden Ansprüche, **dadurch gekennzeichnet,** daß der elektronische Tiefentaster einen resistiven, kapazitiven oder induktiven Wegaufnehmer aufweist.

6. Impressionstonometer nach Anspruch 5 ,**dadurch gekennzeichnet,** daß der Wegaufnehmer ein frequenzbestimmendes Element für einen Schwingkreis bildet.

7. Impressionstonometer nach Anspruch 5 , **dadurch gekennzeichnet,** daß der Wegaufnehmer ein frequenzbestimmendes Element für einen Oszillator (21) bildet.

8. Impressionstonometer nach einem der Ansprüche 6 oder 7 , **gekennzeichnet durch** eine Meßschaltung zur Ermittlung der Frequenz.

9. Impressionstonometer nach Anspruch 8 , **dadurch gekennzeichnet,** daß der Ausgang der Meß schaltung mindestens mittelbar mit dem Eingang einer Anzeigevorrichtung (10) für den Augeninnendruck verbunden ist.

10. Impressionstonometer nach einem der Ansprüche 8 oder 9 , **dadurch gekennzeichnet,** daß die Meßschaltung als Zählschaltung digital arbeitet und das Ausgangssignal mit dem Adresseneingang (25) eines nachgeschalteten Festwertspeichers (26) verbunden ist, wobei in dem Festwertspeicher (26) Datenworte gespeichert sind, welche bei der Anzeigevorrichtung (10) die Anzeige desjenigen Wertes für den Augeninnendruck bewirken, der dem zu der Position des Meßstempels (2) zugeordneten Augeninnendruck entspricht.

11. Impressionstonometer nach Anspruch 5 **dadurch gekennzeichnet,** daß der kapazitive Wegaufnehmer (17) einen mit einer seiner Platten (18) verbundenen Fühlstift (16) aufweist, der unter Federspannung steht und durch Berührung mit dem Meßstempel (2 bzw. 3) des Handgerätes (1) gegen die Wirkung der Feder (19) verschieblich gelagert ist, so daß die beiden Platten (18 und 20) des Kondensators einander angenähert werden.

12. Impressionstonometer nach Anspruch 8 , **dadurch gekennzeichnet,** daß ein dem ersten Festwertspeicher vorgeschalteter, als Latch (26) ausgebildeter erster Zwischenspeicher (24) mit einem, einem zweiten Festwertspeichers (33) vorgeschaltetem als Latch (28) ausgebildeten zweiten Zwischenspeicher derart verbunden ist, daß der zweite Festwertspeicher (28) die der Positionen desjenigen Meßstempels zugeordneten Meßdaten speichert, welcher für die Ermittlung des Augeninnendruckes vorgesehen ist und daß die dem zweiten Meßstempel (3) zugeordneten Meßdaten unter Umgehung des ersten Zwischenspeichers (24) und des ersten Festwertspeichers (26) dem zweiten Zwischenspeicher (28) zugeführt werden, welcher die dem ersten und dem zweiten Meßstempel (2 und 3) zugeordneten Meßdaten dem Adresseneingang (35) des zweiten Festwertspeichers (33) zuführt, wobei in dem zweiten Festwertspeicher (33) Datenworte gespeichert sind, welche bei der Anzeigevorrichtung (11) die Anzeige desjenigen Wertes für die Rigidität des Augapfels bewirken, der der den Positionen der beiden Meßstempel zugeordneten Rigidität entspricht.

13. Impressionstonometer nach einem der vorangehenden Ansprüche, **dadurch gekennzeichnet,** daß die Stromversorgungsschaltung und die Steuerschaltung des ersten Festwertspeichers (26) durch ein mit einem Kontakt (8) versehenen ersten Fühlstift beim Einführen des einen Meßstempels in die Aufnahmevorrichtung (4) aktivierbar ist.

14. Impressionstonometer nach einem der vorangehenden Ansprüche, **dadurch gekennzeichnet,** daß die Stromversorgungsschaltung und die Steuerschaltung des zweiten Festwertspeichers (33) durch ein mit einem Kontakt (8) versehenen ersten Fühlstift und einem einem Kontakt (9) versehenen zweiten Fühlstift beim Einführen des anderen Meßstempels in die Aufnbahmevorrichtung (4) aktivierbar ist, wobei die Kontakte (8 und 9) innerhalb des von dem Handgerät (1) beim Einführen in die Aufnahmevorrichtung (4) zu passierenden Wegquerschnitts gelegen sind.

15. Impressionstonometer nach einem der vorangehenden Ansprüche, **dadurch gekennzeichnet,** daß die Stromversorgungsschalterung ein Zeitglied (36) aufweist, welches die Stromversorgung nach einem vorgegebenen Zeitraum abbricht.

16. Impressionstonometer nach einem der vorangehenden Ansprüche, **dadurch gekennzeichnet,** daß die Anzeigevorrichtung für den Augeninnendruck und/oder die für die Rigidität ein Sieben-Segment-Display aufweist.

17. Impressionstonometer nach einem der vorangehenden Ansprüche, **dadurch gekennzeichnet,** daß die Anzeigevorrichtung für den Augeninnendruck und/oder die für die Rigidität ein LCD-Display aufweist.

18. Impressionstonometer nach einem der vorangehenden Ansprüche, **dadurch gekennzeichnet,** daß als Energiequelle mindestens eine Batterie (38) oder mindestens ein Akkumulator vorgesehen ist.

19. Impressionstonometer nach einem der vorangehenden Ansprüche, **dadurch gekennzeichnet,** daß die Aufnahmevorrichtung (4) als Tischständer, Wandhalterung oder als Kappe entsprechend derjenigen eines Füllfederhalters ausgebildet ist.

20. Impressionstonometer nach einem der vorangehenden Ansprüche, **dadurch gekennzeichnet,** daß das Handgerät (1) aus zwei füllhalterähnlichen Handgeräten, die jeweils verschieden vorgespannte Meßstempel (2 bzw. 3) aufweisen, zusammensetzbar ist.

Fig.1

Fig. 2

Fig. 3

Fig. 4